(19) Europäisches Patentamt
European Patent Office
Office européen des brevets

(11) Veröffentlichungsnummer: **0 103 749**
**B1**

(12) # EUROPÄISCHE PATENTSCHRIFT

(45) Veröffentlichungstag der Patentschrift: **02.07.86**

(21) Anmeldenummer: **83108098.1**

(22) Anmeldetag: **17.08.83**

(51) Int. Cl.⁴: **C 07 D 307/32,** C 07 C 69/757, C 07 C 67/333, C 07 C 69/65, C 07 C 57/76 // C07C53/19, C07C53/50, C07C69/63

(54) **Dialkoxymethyl-butyrolactone, Verfahren zu ihrer Herstellung, Zwischenprodukte dafür und ihre Verwendung.**

(30) Priorität: **26.08.82 DE 3231815**

(43) Veröffentlichungstag der Anmeldung: **28.03.84 Patentblatt 84/13**

(45) Bekanntmachung des Hinweises auf die Patenterteilung: **02.07.86 Patentblatt 86/27**

(84) Benannte Vertragsstaaten: **CH DE FR GB LI**

(56) Entgegenhaltungen: **EP-A-0 031 932**
**DE-B-1 128 067**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Arlt, Dieter, Prof.- Dr., Rybniker Strasse 2, D-5000 Köln 80 (DE)**

EP 0 103 749 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft Dialkoxymethylbutyrolactone, ein Verfahren zu ihrer Herstellung, neue Zwischenprodukte zu ihrer Herstellung und, ihre Verwendung zur Herstellung von Caronaldehydsäurederivaten.

Es sind bereits verschiedene Verfahren zur Herstellung von Caronaldehydsäuren bekannt geworden, z.B. durch partielle Reduktion von Cyclopropandicarbonsäurehalbestern mit Diboran oder Natriumborhydrid zum Hydroxymethyl-cyclopropancarbonsäureester der anschließend mit Chromsäure in Pyridin zu Caronaldeyhdsäure oxidiert wird. Das Verfahren ist jedoch sehr kostspielig, da die verwendeten Ausgangsprodukte nicht leicht zugänglich sind (vgl. DE-OS 2 758 624).

Ein anderes Verfahren geht aus von 4,5-Epoxy-3,3-dimethyl-pentansäureethylester der in absoluten aprotischen Lösungsmitteln mit Litiumdiethylamid zum 2-Hydroxymethyl-3,3-dimethyl-cyclopropancarbonsäureethylester umgesetzt wird, der dann mit Chromsäure in Pyridin zur Caronaldehydsäure oxidiert wird. Auch dieses Verfahren geht von schwer zugänglichen und teuren Ausgangsmaterialien aus (J.H. Babler et. al.,J. Org. Chem. 41 S. 885 ff (1976)).

Es ist weiter bekannt geworden (EP-A-31 932), Caronaldehydsäurederivate (2,2-Dimethyl-3-formyl-(dimethylacetal)-cyclopropancarbonsäuremethylester 9) nach dem im folgenden skizzierten Reaktionsweg zu erhalten:

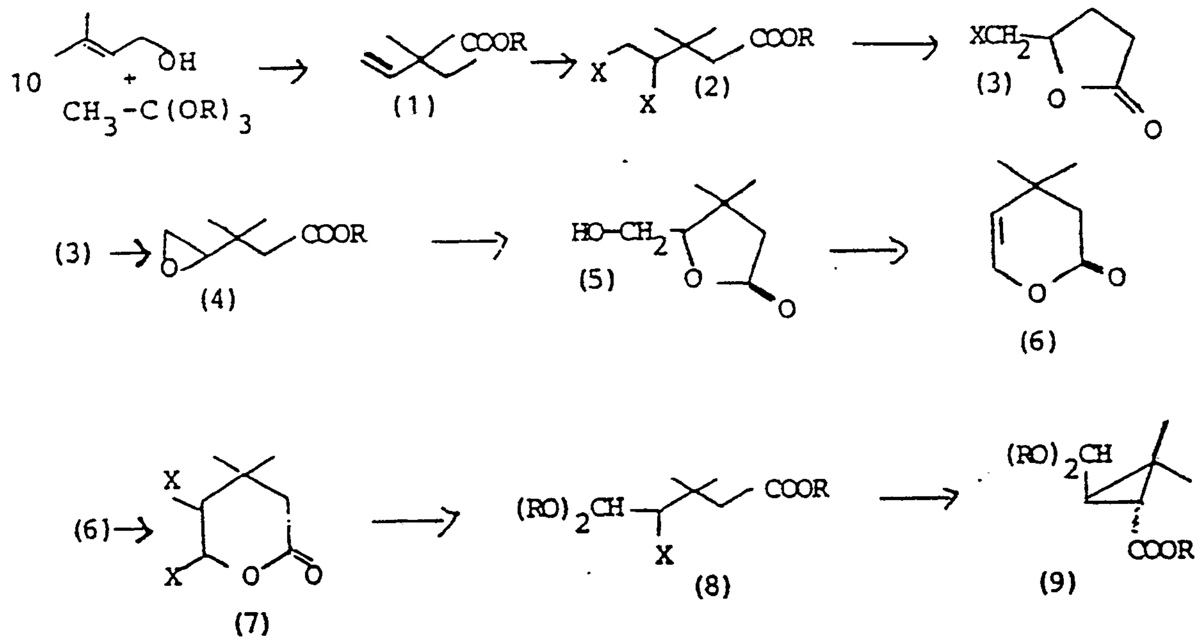

X = Cl o. Br

Schema I

Auch dieses Verfahren hat den Nachteil, daß es von teuren Ausgangsprodukten ausgeht. Außerdem sind, um zu Caronaldehydsäurederivaten (9) zu gelangen 8 Reaktionsstufen erforderlich.

Gegenstand der vorliegenden Anmeldung sind:

1. Dialkoxymethyl-butyrolactone der Formel I

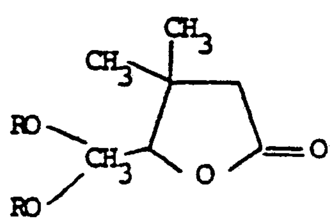

(I)

in welcher

R für $C_{1-4}$-Alkyl steht oder beide Reste R gemeinsam für Ethylen stehen.

2. Verfahren zur Herstellung von Dialkoxymethyl-butyrolactonen der Formel I

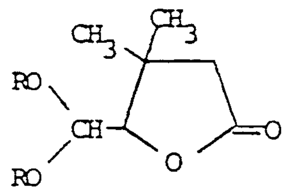

(I)

in welcher
R für $C_{1-4}$-Alkyl steht oder beide Reste R gemeinsam für Ethylen stehen,
dadurch gekennzeichnet, daß man Dichlormethylbutyrolactone der Formel II

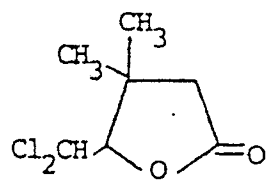

(II)

mit Alkoholaten der Formel III
R - O - M (III)
in welcher
R die oben angegebene Bedeutung hat und
M für ein Äquivalent eines Alkali-oder Erdalkalikations steht,
umsetzt.

3. Verwendung von Dialkoxymethyl-butyrolactonen der Formel I zur Herstellung von Caronaldehydsäurederivaten der Formel IV

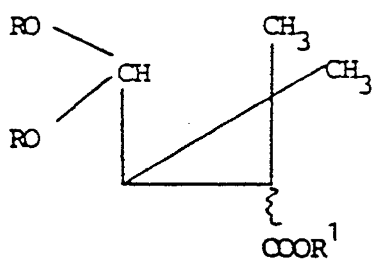

(IV)

in welcher
R die oben angegebene Bedeutung hat und
$R^1$ für $C_{1-4}$-Alkyl steht,
dadurch gekennzeichnet, daß man die Verbindung der Formel I halogenierend spaltet und unter Dreiringbildung dehydrohalogeniert.

4. Dichlormethylbutyrolacton der Formel II

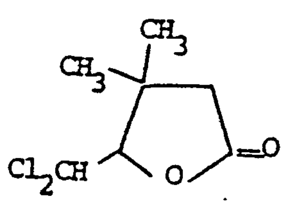

(II)

5. Verfahren zur Herstellung von Dichlormethylbutyrolactonen der Formel II, dadurch gekennzeichnet, daß man 5-Chlor-3,3-dimethyl-pent-4-ensäure der Formel V

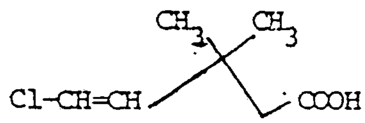

(V)

6. oder ihre Salze (insbesondere Alkali, Erdalkali oder Aminsalze) mit Chlor umsetzt.
5-Chlor-3,3-dimethyl-pent-4-ensäure (derivate) der Formel VI

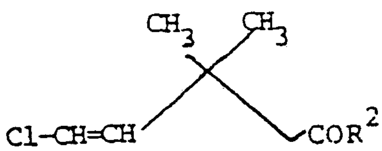

(VI)

in welcher
$R^2$ für OH, $C_{1-4}$-Alkoxy oder Halogen steht.

7. Verfahren zur Herstellung von 5-Chlor-3,3-dimethyl-pent-4-ensäure (derivaten) der Formel VI, dadurch gekennzeichnet, daß man 5,5-Dichlor-3,3-dimethyl -pentansäureester der Formel VII

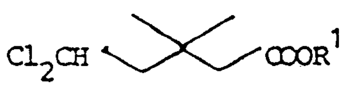

(VII)

in welcher
$R^1$ für $C_{1-4}$-Alkyl steht,
mit Basen umsetzt und die entstandenen Ester der 5-Chlor-3,3-dimethyl-pent-4-en-säure anschließend in üblicher Weise derivatisiert.

Mit Hilfe der neuen Verbindungen und den Verfahren zu ihrer Herstellung wurde ein neues preiswertes Verfahren zur Herstellung von Caronaldehydsäureestern gefunden. Dieses neue Verfahren hat den Vorteil, daß es von einfachen und billigen Ausgangsprodukten ausgeht. So ist das 1, 1, 5, 5-Tetra-chlor-3, 3-dimethyl-pent-1-en, aus dem die 5, 5-Dichlor-3, 3-dimethylpentansäure der Formel VII erhalten wird, in sehr guten Ausbeuten über zwei Stufen aus Tetrachlorkohlenstoff, iso-Buten und Vinylchlorid zugänglich. Die weitere Umsetzung zum Caronaldehydsäurester erfolgt in 5 Stufen. Das heißt verglichen mit dem aus dem Stand der Technik bekannten Verfahren ist eine Stufe weniger zu durchlaufen.

Es war überraschend, daß mit Hilfe der neuen Verbindungen das Gesamtverfahren zur Herstellung der Caronaldehydsäureester durchgeführt werden konnte. Die Vorteile des neuen Verfahrens liegen darin, daß billigere Ausgangsprodukte verwendet werden können und daß insgesamt eine Reaktionsstufe weniger benötigt wird um zum Caronaldehydsäureester zu gelangen.

Der Verlauf der Gesamtreaktion war nicht nur deshalb überraschend, weil zu seiner Durchführung neue Verbindungen eingesetzt werden mußten, über deren Eigenschaften und Reaktionsfähigkeiten noch nichts bekannt war. Es war auch überraschend, daß verschiedene der durchlaufenden Reaktionsschritte überhaupt in der beschriebenen Weise abliefen.

So konnte nicht erwartet werden, daß das Verfahren unter 5 (oben) glatt und mit guten Ausbeuten verläuft. Es hätte erwartet werden können, daß durch Halogenaddition an die Doppelbindung und durch Bildung eines 6-Ringlactons anstelle des gewünschten Butyrolactons Ausbeute und Reinheit der Verbindung der Formel II so schlecht sind, daß das Gesamtverfahren nicht wirtschaftlich durchzuführen ist.

So konnte auch nicht erwartet werden, daß das Verfahren unter 2 (oben) glatt und mit guten Ausbeuten verläuft. Auch hier mußte mit Nebenreaktionen (HCl-Eliminierung unter Ausbeute einer Doppelbindung) gerechnet werden, wobei Ausbeute und Reinheit das Verfahren unwirtschaftlich hätten machen können.

Die Dialkoxymethyl-butyrolactone der Formel I sind neu. In Formel I steht R bevrozugt für Methyl oder Ethyl. Sie werden nach dem unter 2 (oben) angegebenen Verfahren hergestellt. Dies kann, wenn Dichlormethylbutyrolacton und Natriummethylat verwendet wird durch folgendes Formelschema wiedergegeben werden:

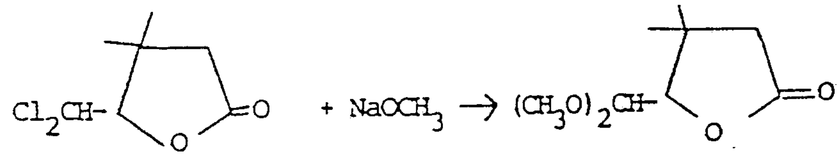

Die Reaktion wird zwischen 20 und 100° C bevorzugt zwischen 20 und 80° C durchgeführt. Man arbeitet im allgemeinen bei Normaldruck. Die Reaktion wird in inerten Lösungsmitteln durchgeführt. Bevorzugt sind die den Alkoholaten zugrundeliegenden Alkohole als Lösungsmittel, also z.B. Methanol.

Die Verbindungen der Formel I können in üblicher Weise aus dem Reaktionsgemisch durch Destillation isoliert werden. Sie können aber auch in Form des Reaktionsgemisches sofort, ohne Isolierung, zu den Verbindungen der Formel IV weiter umgesetzt werden. Dazu wird Chlorwasserstoff in das Reaktionsgemisch, das bei der Bildung der Verbindungen der Formel I entstand, eingeleitet und nach Beendigung der Reaktion das Gemisch mit Alkalialkoholaten wie Natriummethylat oder Natriumethylat versetzt.

Verbindungen der Formel IV werden auch erhalten, wenn Butyrolactone der Formel I mit Thionylchlorid oder Phosphorpentachlorid z.B. bei Temperaturen zwischen 0° und 40°C umgesetzt werden und das erhaltene Produkt mit Alkoholen und basischen Mitteln behandelt wird. Als Alkohole kommen z.B. Methanol und Ethanol, als basische Mittel z.B. Na-methylat und -ethylat oder DBU bzw. DBN in Frage.

Die Isolierung der Butyrolactone der Formel I erfolgt in an sich bekannter Weise z.B. durch Destillation.

Das Dichlormethyl-butyrolacton der Formel II ist neu. Es wird nach folgendem Formelschema erhalten

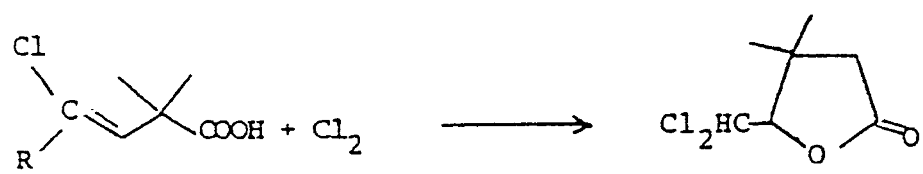

Die Reaktion erfolgt durch Umsetzung der Säure der Formel V oder von Salzen dieser Säure mit Chlor zu dem 5-Dichloro-methyl-4, 4-dimethyl-butyrolacton der Formel II. Die Umsetzung erfolgt bei Temperaturen zwischen ca. -20° und 50°C in inerten Lösungsmitteln, wie z.B. Dichlor- oder Trichlormethan. Als Salze eignen sich die Alkalisalze der Säure der Formel V, aber auch deren Salze mit tertiären Basen, wie z.B. Trimethyl- oder Triethylamin sind verwendbar. Das Lacton der Formel II kann durch fraktionierende Destillation rein isoliert werden, gegebenenfalls nachdem zuvor die entstandenen Salze, z.B. Triethylammoniumchlorid, oder gebildeter Chlorwasserstoff aus dem Reaktionsgemisch mit Wasser extrahiert wurden.

5-Chlor-3, 3-dimethyl-pent-4-ensäure und ihre Derivate der Formel V und VI sind neu.

Sie wird nach folgendem Formelschema erhalten:

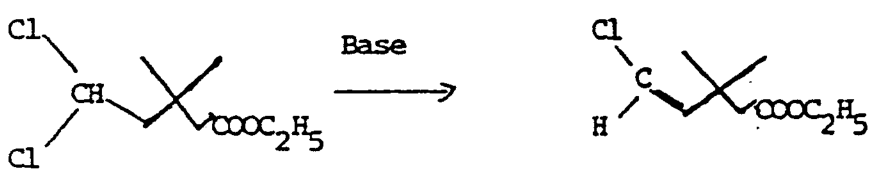


Durch übliche Verseifung des Esters werden die Säure der Formel V bzw. ihre Salze erhalten.

Der Ester der 5, 5-Dichlor-3, 3-dimethyl-pentansäure wird durch Einwirkung von Basen dehydrohalogeniert, wobei Ester der 5-Chlor-3, 3-dimethyl-pent-4-ensäure der Formel V erhalten werden. Als Dehydrohalogenierungsmittel werden z.B. Alkoholate, wie Na-methylat, -ethylat und -butylat oder tertiäre Basen wie Triethylamin, N,N-Dimethylbenzylamin, DBU und DBN verwendet. Die Dehydrohalogenierung wird im allgemeinen bei Temperaturen zwischen 65° C und 150° C vorgenommen, als Lösungsmittel dienen z.B. Alkohole wie Methanol, Ethanol und Butanol oder aprotische Lösungsmittel wie Dimethylformamid und Diethylenglykoldimethylether. Der erhaltene Ester kann z.B. durch Destillation rein isoliert werden. Er kann aber auch in situ durch Umsetzung mit Alkalien hydrolysiert werden. Als Alkalien dienen z.B. Natrium- oder Kaliumhydroxid. Diese Esterhydrolyse wird z.B. in Alkoholen bei Temperaturen zwischen 20° und 80° vorgenommen. Durch Ansäuren mit Mineralsäuren wie Salzsäure oder Schwefelsäure wird die 5-Chlor-3, 3-dimethyl-pent-4-ensäure anschließend freigesetzt und z.B. durch Destillation in reiner Form erhalten.

Es ist auch möglich, das Chlorid der Säure der Formel V durch katalytische Dehydrohalogenierung aus dem 5,5-Dichlor-3,3-dimethyl-pentansäurechlorid zu erhalten. Als Katalysatoren eignen sich z.B. mit Metallsalzen dotierte Aktivkohle, wobei als Salze z.B. $BaCl_2$, $FeCl_3$, $ZnCl_2$ und CuCl verwendet werden können. Die Dehydrohalogenierung, die sowohl in der Flüssigphase wie in der Gasphase erfolgen kann, wird bei Temperaturen zwischen 150° und 300°C ausgeführt. Das erhaltent Säurechlorid der Säure der Formel V wird durch Destillation gereinigt. Es kann in an sich bekannter Weise zur Säure der Formel V hydrolysiert werden.

5, 5-Dichlor-3, 3-dimethylpentansäure der Formel VII ist Gegenstand einer noch nicht zum Stand der Technik gehörenden Anmeldung der Anmelderin (vgl. EP-A- 103 136)

Man erhält sie durch saure Hydrolyse von 1, 1, 5, 5-Tetra-chlor-3, 3-dimethyl-pent-1-en, beispielsweise mit Schwefelsäure. Während der Hydrolyse wird ein Temperaturbereich von ca. 20°-100°C, vorzugsweise von 40°-70°C, eingehalten. Im allgemeinen wird ein Überschuß an Säure eingesetzt, mindestens jedoch soll das Reaktionsgemisch die dem eingesetzten Penten äquivalente Menge Wasser ent halten. Zur Hydrolyse sind neben Schwefelsäure auch andere starke Säuren wie z.B. Phosphorsäure, Methansulfonsäure, Trifluoressigsäure und Gemische aus Ameisensäure und Salzsäure geeignet.

Will man die Säure der Formel VII isolieren, so wird das Reaktionsgemisch mit Wasser verdünnt und die Säure der Formel VII mit Lösungsmitteln, wie z.B. Chlorkohlenwasserstoffen extrahiert. Bei Verwendung destillierbarer Säuregemische, wie z.B. die nach der Hydrolyse mit Trifluoressigsäure oder Ameisensäure-Salzsäure erhaltenen Reaktionsgemische, werden destillativ aufgearbeitet, um die Säure der Formel VII rein zu gewinnen.

Es ist aber auch möglich, die nach der sauren Hydrolyse erhaltenen Reaktionsgemische direkt weiter umzusetzen (vgl. Verfahren 7).

Um 5-Chlor-3, 3-dimethyl-pent-4-ensäure der Formel V zu erhalten, wird die Verbindung der Formel VII zunächst in einen Ester umgewandelt. Dies kann z.B. in bekannter Weise durch Umsetzung mit Thionylchlorid oder Phosgen zum 5,5-Dichlor-3, 3-dimethyl-pentansäurechlorid und die anschließende Umsetzung dieser Verbindung mit Alkoholen, wie z.B. Methanol oder Ethanol, erfolgen. Es ist auch möglich, die Säure in Gegenwart von bekannten Veresterungskatalysatoren direkt mit Alkoholen umzusetzen, wobei zweckmäßig das entstehende Reaktionswasser durch Azeotropdestillation entfernt wird. Besonders vorteilhaft ist für die Herstellung des Esters der Säure der Formel VII die Umsetzung der aus 1, 1, 5, 5-Tetrachlor-3, 3-dimethyl-pent-1-en erhaltenen Reaktionsgemische mit Alkoholen. Der gebildete Ester wird in diesem Fall z.B. durch Extraktion mit Lösungsmitteln wie z. B. Hexan gegebenenfalls nach Verdünnen des Reaktionsgemisches mit Wasser isoliert und durch Destillation gereinigt.

1, 1, 5, 5-Tetrachlor-3, 3-dimethyl-pent-1-en ist bekannt. Es ist preiswert aus Tetrachlorkohlenstoff, iso-Buten und Vinylchlorid nach an sich bekannten Verfahren zugänglich.

**Beispiel 1**

5, 5-Dichlor-3, 3-dimethyl-pentansäure

500 g 1, 1, 5, 5-Tetrachlor-3, 3-dimethyl-penten werden innerhalb 1 Stunde unter Rühren so zudosiert, daß die Reaktionsmischung nicht über 45°C steigt. Das Reaktionsgemisch wird weitere 24 Stunden bei 40°-45°C gehalten und dann auf 4 kg Eis gegeben. Die Mischung wird mehrfach mit Dichlormethan extrahiert. Nach dem Abdestillieren des Lösungsmittels erhält man 420 g rohe 5, 5-Dichlor-3, 3-di-methyl-pentansäure, nach der Destillation 380 g reine 5, 5-Dichlor-3,3-dimethyl-pentansäure, $Kp._{0,2}$ 103-106°C.

**Beispiel 2**

5, 5-Dichlor-3, 3-dimethyl-pentansäurechlorid

52, 25 g 5, 5-Dichlor-3, 3-dimethyl-pentansäure werden mit 150 ml Thionylchlorid zunächst bei Raumtemperatur, nachfolgend unter Erwärmen auf 60°C umgesetzt. Durch destillative Aufarbeitung erhält man 56,5 g 5,5-Dichlor-3,3 -dimethyl-pentansäurechlorid, $Kp._{15}$ 114-115°C.

**Beispiel 3**

5, 5-Dichlor-3, 3-dimethyl-pentansäuremethylester

22, 5 g des nach Beispiel 2 erhaltenen Säurechlorids werden zu 100 ml Methanol zugetropft und anschließend 1 Std. auf 65°C erwärmt. Durch Destillation des Reaktionsgemisches erhält man 20, 5 g 5, 5-Dichlor-3, 3-dimethyl-pentansäuremethylester, $Kp._{15}$ 110°-112°C.

**Beispiel 4**

5-Chlor-3, 3-dimethyl-pent-4-ensäuremethylester

62, 8 g des nach Beispiel 3 erhaltenen Esters und 40, 5 g DBN (Diazabicyclononan) werden in 250 ml Dimethoxyethan 40 Stunden auf Rückflußtemperatur erwärmt.

Dann wird das Lösungsmittel abdestilliert und der Rückstand mit 250 ml Dichlormethan versetzt und mit verdünnter Salzsäure extrahiert. Durch Destillation der organischen Phase werden 30 g 5-Chlor-3, 3-dimethyl-pent-4-en-säuremethylester, $Kp._{12}$ 87-88° C, erhalten.

**Beispiel 5**

5-Chlor-3, 3-dimethyl-pent-4-ensäurechlorid

38 g des nach Beispiel 4 erhaltenen Esters werden mit 13 g KOH, gelöst in 120 ml Methanol, 8 Stunden auf 40° C erwärmt. Dann wird das Methanol abdestilliert und der Rückstand mit 150 ml Wasser aufgenommen und die Mischung mit Salzsäure angesäuert. Man extrahiert anschließend mit Dichlormethan und verdampft das Lösungsmittel im Vakuum. Die zurückbleibende Säure wird mit 100 ml Thionylchlorid versetzt und 4 Stunden auf 60° C erwärmt. Nach der Destillation des Reaktionsgemisches erhält man 36 g 5-Chlor-3, 3-dimethyl-pent-4-ensäurechlorid, $Kp._{15}$ 86°-90° C.

**Beispiel 6**

4-Dichlormethyl-3, 3-dimethyl-butyrolacton

13, 1 g des nach Beispiel 5 erhaltenen Säurechlorids werden zu einer gerührten Mischung von 4 g Wasser in 150 ml Dichlormethan getropft. Die Mischung wird 8 Stunden auf Rückflußtemperatur erhitzt. Anschließend werden das Lösungsmittel, Restwasser und Chlorwasserstoff im Vakuum entfernt. Der Rückstand (5-Chlor-3, 3-dimethyl-pent-4-ensäure) wird in 50 ml Dichlormethan gelöst.

Zu dieser Lösung werden 9, 2 g Triethylamin zugegeben und anschließend werden in diese Salzlösung ca. 7 g Chlor unter Kühlung auf 0° C eingeleitet.

Um das gebildete 4-Dichlormethyl-3, 3-dimethyl -butyrolacton zu isolieren, wird die erhaltene Lösung mit Wasser extrahiert, getrocknet ($Na_2SO_4$) und destilliert. Man erhält 8, 5 g 4-Dichlormethyl-3, 3-dimethyl-butyrolacton, $Kp._{0,15}$ 88°-92° C.

Zweckmäßig wird das erhaltene rohe Reaktionsprodukt in Lösung weiter gemäß Beispiel 7 umgesetzt.

**Beispiel 7**

Die nach Beispiel 6 erhaltene Dichlormethanlösung von 4-Dichlormethyl-3, 3-dimethyl-butyrolacton wird zu einer Lösung von 6, 5 g Na in 100 ml absol. Ethanol bei 0° C zugetropft. Dann wird auf Rückflußtemperatur erwärmt und über eine Kolonne das Dichlormethan abdestilliert. Nach 6 Stunden wird die Reaktionslösung mit Eiswasser versetzt und mit Dichlormethan mehrfach extrahiert. Die fraktionierte Destillation der Dichlormethanlösung ergibt 8,9 g 4-Diethoxymethyl -3,3-dimethyl-butyrolacton, $Kp._{0,08}$ 78°-80° C.

**Beispiel 8**

8, 9 g des nach Beispiel 7 erhaltenen 4-Diethoxymethyl-3, 3-dimethyl-butyrolactons werden in 80 ml absol. Ethanol gelöst und bei Raumtemperatur wird Chlorwasserstoff in die Lösung eingeleitet. Nach 10 Stunden wird das Lösungsmittel und überschüssiger Chlorwasserstoff im Vakuum verdampft. Der Rückstand wird in die Lösung von 1 g Na in 100 ml Ethanol eingetropft und 5 Stunden auf 75° C erwärmt. Das Reaktionsgemisch wird nach Filtration des ausgefallenen Natriumchlorids destilliert. Man erhält 5 g Caronaldehydsäureethylester-diethylacetat, $Kp_{0,04}$ 73° C-77° C.

**Patentansprüche**

1. Dialkoxymethyl-butyrolactone der Formel I

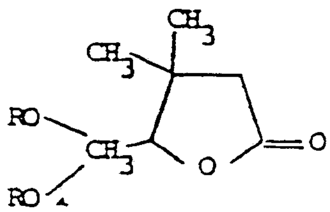

(I)

in welcher

R für $C_{1-4}$-Alkyl steht oder beide Reste R gemeinsam für Ethylen stehen.

2. Verfahren zur Herstellung von Dialkoxymethyl-butyrolactonen der Formel I

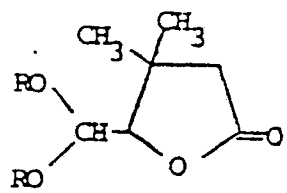

(I)

in welcher

R für $C_{1-4}$-Alkyl steht oder beide Reste R gemeinsam für Ethylen stehen,

dadurch gekennzeichnet, daß man Dichlormethylbutyrolactone der Formel II

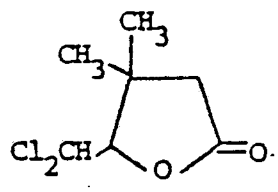

(II)

mit Alkoholaten der Formel III

R - O - M (III)

in welcher

R die oben angegebene Bedeutung hat und

M für ein Äquivalent eines Alkali-oder Erdalkalikations steht,

umsetzt.

3. Verwendung von Dialkoxymethyl-butyrolactonen der Formel I zur Herstellung von Caronaldehydsäurederivaten der Formel IV

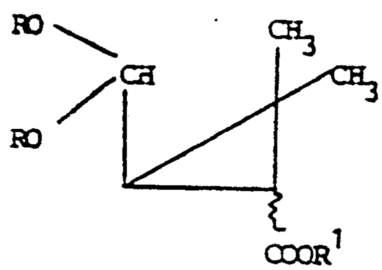

(IV)

in welcher

R, die im Anspruch 1 angegebene Bedeutung hat und

$R^1$ für $C_{1-4}$-Alkyl steht,

dadurch gekennzeichnet, daß man die Verbindung der Formel I halogenierend spaltet und unter Dreiringbildung dehydrohalogeniert.

4. Dichlormethylbutyrolacton der Formel II

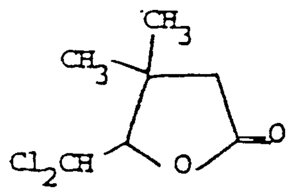

(II)

5. Verfahren zur Herstellung von Dichlormethylbutyrolactonen der Formel II, dadurch gekennzeichnet, daß man 5-Chlor-3,3-dimethyl-pent-4-ensäure der Formel V

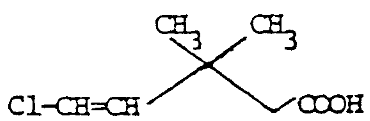

(V)

oder ihre Salze (insbesondere Alkali, Erdalkali oder Aminsalze) mit Chlor umsetzt.

6. 5-Chlor-3,3-dimethyl-pent-4-ensäure (derivate) der Formel VI

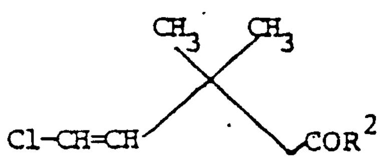

(VI)

in welcher

$R^2$ für OH, $C_{1-4}$-Alkoxy oder Halogen steht.

7. Verfahren zur Herstellung von 5-Chlor-3,3-dimethyl-pent-4-ensäure (derivaten) der Formel VI, dadurch gekennzeichnet, daß man 5,5-Dichlor-3,3-dimethyl-pen-tansäureester der Formel VII

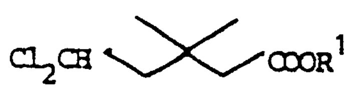

(VII)

in welcher

$R^1$ für $C_{1-4}$-Alkyl steht,

mit Basen umsetzt und die entstandenen Ester der 5-Chlor-3,3-dimethyl-pent-4-en-säure anschließend in üblicher Weise derivatisiert.

**Claims**

1. Dialkoxymethyl-butyrolactones of the formula I

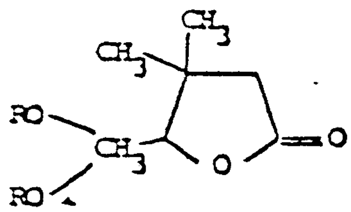

(I)

in which
R represents $C_{1-4}$-alkyl, or both radicals R together represent ethylene.

2. Process for the preparation of dialkoxymethylbutyrolactones of the formula I

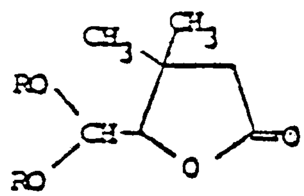

(I)

in which
R represents $C_{1-4}$-alkyl, or both radicals R together represent ethylene,
characterised in that dichloromethylbutyrolactones of the formula II

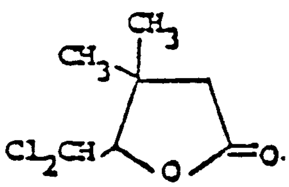

(II)

are reacted with elcoholatee of the formula
R - O - M (III)
in which
R has the meaning given above and
M represents one equivalent of an alkali metal or alkaline earth metal cation.

3. Use of dialkoxymethyl-buryrolactones of the formula I for the preparation of caronaldehydic acid derivatives of the formula IV

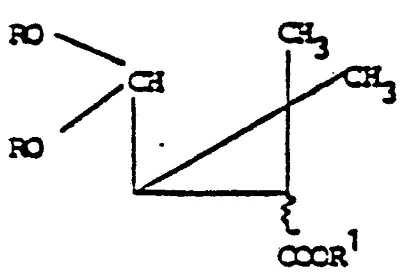

(IV)

in which
R has the meaning given in Claim 1 and
$R^1$ represents $C_{1-4}$-alkyl,
characterised in that the compound of the formula I is cleaved by halogenation, and the product is dehydrohalogenated, with the formation of a three-membered ring.

4. Dichloromethylbutyrolactone of the formula II

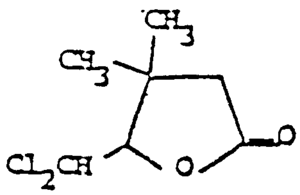

(II)

5. Process for the preparation of dichloromethylbutyrolactones of the formula II, characterised in that 5-chloro-3,3-dimethyl-pent-4-enoic acid of the formula

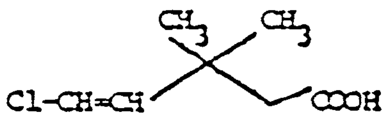

(V)

or its salts (in particular alkali metal, alkaline earth metal or amine salts), are reacted with chlorine.

6. 5-Chloro-3,3-dimethyl-pent-4-enoic acid (derivatives) of the formula VI

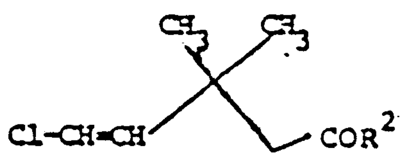

(VI)

in which
$R^2$ represents OH, $C_{1-4}$-alkoxy or halogen.

7. Process for the preparation of 5-chloro-3,3-dimethyl-pent-4-enoic acid (derivatives) of the formula VI, characterised in that 5,5-dichloro-3,3-dimethyl-penta-noic acid esters of the formula VIII

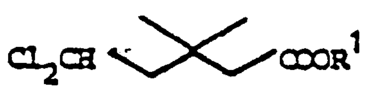

(VII)

in which
$R^1$ represents $C_{1-4}$-alkyl, are reacted with bases, and the resulting esters of 5-chloro-3,3-dimethyl-pent-4-enoic acid are then derivatised in a costumary manner.

**Revendications**

1. Dialkoxyméthyl-butyrolactones de formule I

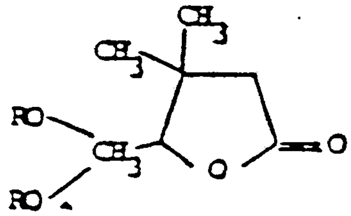

(I).

dans laquelle
R désigne un groupe alkyle en $C_1$ à $C_4$ ou bien les deux restes R forment conjointement un groupe éthylène.

2. Procédé de production de dialkoxyméthylbutyrolactones de formule II

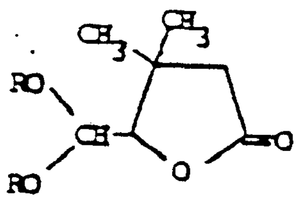

(I)

dans laquelle
R représente un groupe alkyle en $C_1$ à $C_4$ ou bien les deux restes R forment ensemble un groupe éthylène, caractérisé en ce qu'on fait réagir des dichlorométhylburyrolactones de formule

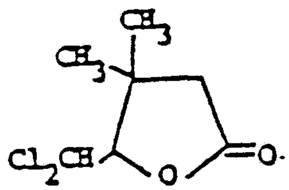

(II)

avec des alcoolates de formule III
R - O - M (III)
dans laquelle
R a la définition indiquée ci-dessus et
M est un équivalent d'un cation de métal alcalin ou de métal alcalino-terreux.

3. Utilisation de dialkoxyméthyl-butyrolactones de formule I pour la préparation de dérivés d'acide caronaldéhydique de formule IV

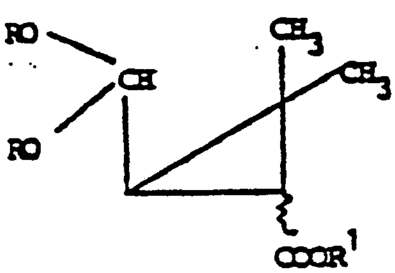

(IV)

dans laquelle
R a la définition indiquée dans la revendication 1 et
$R^1$ représente un groupe alkyle en $C_1$ à $C_4$, caractérisée en ce qu'on effectue la coupure par halogénation du composé de formule I et sa déshydrohalogénation avec formation d'un noyau triangulaire.

4. La dichlorométhylbutyrolactone de formule II

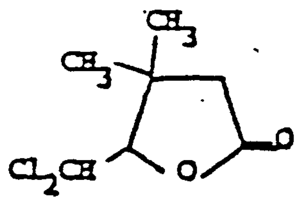

(II)

5. Procédé de production de dichlorométhylbutyrolactones de formule II, caractérisé en ce qu'on fait réagir l'acide 5-chloro-3,3-diméthylpent-4-énoïque de formule V

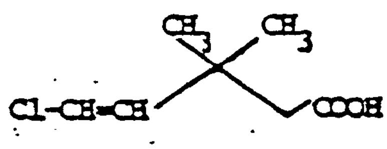

(V)

ou ses sels (notamment ses sels de métaux alcalins, de métaux alcalino-terreux ou d'amines) avec du chlore.

6. Acide 5-chloro-3,3-diméthylpent-4-énoïque (dérivés) de formule VI

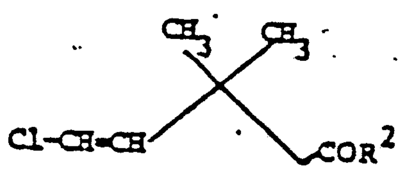

(VI)

dans laquelle

$R^2$ représente OH, un groupe alkoxy en $C_1$ à $C_4$ ou un halogène.

7. Procédé de production de l'acide 5-chloro-3,3-diméthylpent-4-énoïque (dérivés) de formule VI, caractérisé en ce qu'on fait réagir un ester d'acide 5,5-dichloro-3,3-diméthylpentanoïque de formule VII

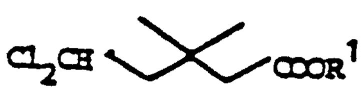

(VII)

dans laquelle

$R^1$ est un groupe alkyle en $C_1$ à $C_4$, avec des bases, puis on transforme en dérivés de manière classique les esters formés de l'acide 5-chloro-3,3-di-méthylpent-4-énoïque.